# EUROPEAN PATENT APPLICATION

(11) **EP 4 635 431 A1**
(43) Date of publication of application: **22.10.2025**
(21) Application number: 23902376.5
(22) Date of filing: 09.11.2023
(51) Int. Cl.: A61B 17/34

(54) **VARIABLE-DIAMETER TISSUE EXPANSION DEVICE**

(30) Priority: 13.12.2022 CN 202211629216
(71) Applicant: Beijing Taijieweiye Technology Co., Ltd., Beijing 101200 (CN)
(72) Inventor: LI, Kai, Beijing 101200 (CN); CUI, Wei, Beijing 101200 (CN); XU, Yongsong, Beijing 101200 (CN); TANG, Hang, Beijing 101200 (CN); WU, Jian, Beijing 101200 (CN); QIN, Chuan, Beijing 101200 (CN); FU, Peiyun, Beijing 101200 (CN); DUAN, Hualei, Beijing 101200 (CN)
(74) Representative: Stanners, David Ralph
(86) International application number: PCT/CN2023/130841
(87) International publication number: WO 2024/125176

(57) **Abstract**

The present invention belongs to the field of surgical equipment technology, and specifically relates to a variable diameter tissue expansion device, which includes an operating frame, a pushing block, support rods and an expansion tube; both the operating frame and the pushing block are provided with a central cavity. A plurality of the support rods are distributed in a circular array around the cavity on the operating frame, the support rods comprise the first sliding part, the second sliding part and an expansion part, and the first sliding part is in the sliding fit with the operating frame, the second sliding part is in the sliding fit with the pushing block, and the pushing block is in the sliding fit with the operating frame; a plurality of expansion parts are supported on the inner wall of the expansion tube, the angle between the expansion part and the first sliding part is a right angle, and the angle between the expansion part and the second sliding part is an obtuse angle, so that the diameter of the expansion tube can be expanded during sliding. When the expansion tube enters the brain tissue, it is in a contracted state and causes little damage to the brain tissue; when the expansion tube expands, it squeezes the brain tissue more evenly, slowly, and without friction, which can better protect the brain tissue.

## Description

### Technical field

The present invention belongs to the field of surgical equipment technology, specifically, the present invention relates to a variable diameter tissue expansion device.

### Background technique

Currently, neurosurgical clinical doctors widely use self-made sheath tubes to establish channels for surgical procedures. The sheath is usually a polymer or glass tube used as a tissue expander, lined with an expansion tube. When establishing a channel, the expansion tube is inserted into the brain tissue with the tissue expander to reach the lesion site. After removing the expansion tube, the tissue expander is fixed, and instruments such as endoscope, drainage tube, surgical forceps, bipolar electrocoagulation, etc. are used to reach the lesion site through the inner cavity of the tissue expander for surgical operation.

At present, the tissue expanders are a constant diameter polymer or glass tube used as a channel for surgical operations. The tissue expander has a large outer diameter, which causes significant compression and friction scratches on the brain tissue during insertion into the brain tissue, resulting in great impact and damage to the brain tissue.

### Contents of the invention

The present invention provides a variable diameter tissue expansion device, which can solve the technical problem of brain tissue damage caused by tissue expanders in the prior art.

In order to solve the above problems, the present invention provides a variable diameter tissue expansion device, and its technical solution is as follows:

A variable diameter tissue expansion device comprising an operating frame, a pushing block, support rods, and an expansion tube; the operating frame and the pushing block are both equipped with a central cavity; a plurality of support rods are distributed in a circular array around the cavity on the operating frame, the support rods comprise the first sliding part, the second sliding part and an expansion part, and the first sliding part is in the sliding fit with the operating frame, the second sliding part is in the sliding fit with the pushing block, and the pushing block is in the sliding fit with the operating frame; a plurality of expansion parts are supported on the inner wall of the expansion tube, the angle between the expansion part and the first sliding part is a right angle, and the angle between the expansion part and the second sliding part is an obtuse angle, so that the diameter of the expansion tube can be expanded during sliding.

The variable diameter tissue expansion device as described above is further preferred to include an elastic body, which is installed on the operating frame and located between the operating frame and the pushing block; one end of the elastic body is in contact with the operating frame, and another end is in contact with the pushing block, used to provide reset thrust for the pushing block.

The variable diameter tissue expansion device as described above is further preferred, wherein the elastic body is either a pressure spring or a rubber block, or a combination thereof.

The variable diameter tissue expansion device as described above is further preferred to include an adjusting knob, the adjusting knob is provided with a central cavity; the adjusting knob is installed on the operating frame and is in contact with the pushing block, which is located between the adjusting knob and the elastic body; the adjusting knob is threaded with the operating frame to push the pushing block to slide when rotated.

The variable diameter tissue expansion device as described above is further preferred to have anti slip patterns on the circumference of the adjusting knob.

The variable diameter tissue expansion device as described above is further preferred as follows: the operating frame comprises the first operating block connectively and the second operating block connectively; the first operating block is equipped with limiting position groove, which is multiple; multiple limiting position grooves correspond one-to-one with multiple expansion parts, used to limit the swing of the expansion part when it reaches its maximum position.

The variable diameter tissue expansion device as described above is further preferred as follows: the second operating block is provided with the first sliding groove that slides and fits with the first sliding part, and the first sliding groove is located on the bottom surface of the second operating block; along the axial direction of the expansion tube, the depth of the first sliding groove is less than the height of the first sliding part; the first operating block and the second operating block are connected by threads, and there is an adjustable gap between the first operating block and the second operating block, the adjustable gap is used to cooperate with the first sliding groove to slide or lock the first sliding part.

The variable diameter tissue expansion device as described above is further preferred as follows: the second operating block is provided with a first sliding groove that slides and fits with the first sliding part, and the first sliding groove is located on the bottom surface of the second operating block; along the axial direction of the expansion tube, the depth of the first sliding groove is less than the height of the first sliding part; the first operating block and the second operating block are connected by snap-in connection, and are provided with the first snap-in connection position and the second snap-in connection position, the first operating block and the second operating block are provided with the first gap when the first the second snap-in connection position is engaged, the first operating block and the second operating block are provided with the second gap when the second snap-in connection position is engaged; the sum of the depth values of the first gap and the first sliding groove is greater than the height value of the first sliding part, and the sum of the depth values of the second gap and the first sliding groove is less than the height value of the first sliding part.

The variable diameter tissue expansion device as described above is further preferred as follows: the operating frame is equipped with guide bars, and the pushing block is equipped with guide grooves, the guide bars slide and fit with the guide groove to provide sliding guidance for the pushing block.

The variable diameter tissue expansion device as described above is further preferred as follows: the length of the guide groove is greater than the length of the guide bars.

The variable diameter tissue expansion device as described above is further preferred as follows: the guide grooves and the guide bars are both multiple, and multiple guide bars correspond one-to-one with multiple guide grooves.

The variable diameter tissue expansion device as described above is further preferred as follows: the top of the pushing block is funnel-shaped, with a sloping surface and a fitting edge; the sloping surface gradually slopes downwards from the periphery of the pushing block towards the middle; the fitting edge is set at the periphery of the top of the pushing block, located on the outer side of the sloping surface.

The variable diameter tissue expansion device as described above is further preferred as follows: the top surface of the mating edge is a circular arc surface, used to press against the adjusting knob.

The variable diameter tissue expansion device as described above is further preferred as follows: the pushing block is equipped with the second sliding groove that slides and fits with the second sliding part, the second sliding grooves are multiple, and multiple second sliding grooves correspond one-to-one with multiple second sliding parts.

The variable diameter tissue expansion device as described above is further preferred as follows: the length of the second sliding groove is greater than the length of the second sliding part.

The variable diameter tissue expansion device as described above is further preferred as follows: the bottom end of the expansion part is provided with an outwardly curved snap-in protrusion, the snap-in protrusion protrudes from the outer surface of the expansion part to prevent the expansion tube from detaching from the support rods.

The variable diameter tissue expansion device as described above is further preferred as follows: along the axial direction of the expansion tube, the top outer diameter of the expansion tube is set to expand outward..

Analysis shows that compared with the prior art, the advantages and beneficial effects of the present invention are:

The present invention provides a variable diameter tissue expansion device, which is suitable for neurosurgery to establish subcortical passages. The expansion tube has elasticity and enters the brain tissue in a contracted state with a small outer diameter, thus reducing the extent of damage to the cerebellar tissue during insertion; when the expansion tube expands radially, the compression of brain tissue in the circumferential direction is more uniform and slow, without axial frictional resistance, which can better protect brain tissue.

### Description of drawings

Fig. 1 is a schematic structural diagram of the variable diameter tissue expansion device of the present invention;
Fig. 2 is a cross-sectional view of the variable diameter tissue expansion device of the present invention;
Fig. 3 is a schematic diagram of the connection between the operating frame and the pushing block of the present invention;
Fig. 4 is a schematic structural diagram of the pushing block of the present invention;
Fig. 5 is a cross-sectional view of the pushing block of the present invention;
Fig. 6 is a schematic structural diagram 1 of the operating frame of the present invention;
Fig. 7 is a schematic structural diagram 2 of the operating frame of the present invention;
Fig. 8 is a cross-sectional view of the operating frame of the present invention;
Fig. 9 is a schematic structural diagram of the expansion tube of the present invention;
Fig. 10 is a schematic diagram of the distribution of multiple support rods of the present invention;
Fig. 11 is a schematic structural diagram of the support rods of the present invention.

In the picture: 1-Adjusting knob; 2-Operating frame; 3-Expansion tube; 4-Support rods; 5-Pushing block; 6-Elastic body; 7-Slope surface; 8-Fitting edge; 9-Guide groove; 10-The second sliding groove; 11-The second operating block; 12-guide bar; 13-The first sliding groove; 14-limiting groove; 15-The first operating block; 16-The second sliding part; 17-The first sliding part; 18-Expansion part; 19 -Snap-in protrusion.

### Detailed Description

The following will provide a clear and complete description of the technical solution in the embodiments of the present invention, in conjunction with the attached figures, obviously, the described embodiments are only a part of the embodiments of the present invention, not all embodiments. Based on the embodiments of the present invention, all other embodiments obtained by ordinary skilled persons in the art without creative labor are within the scope of protection of the present invention.

In the description of the present invention, the terms "longitudinal", "transverse", "up", "down", "front", "back", "left", "right", "vertical", "horizontal", "top", "bottom", etc. indicate orientation or positional relationships based on the orientation or positional relationships shown in the attached figures, only for the convenience of describing the present invention and not to require the present invention to be constructed and operated in a specific orientation, and therefore cannot be understood as limitations on the present invention. The terms "linked" and "connected" used in the present invention should be broadly understood, for example, it can be fixed connections or detachable connections; It can be directly connected or indirectly connected through intermediate components. For ordinary technical personnel in this field, the specific meaning of the above terms can be understood according to the specific situation.

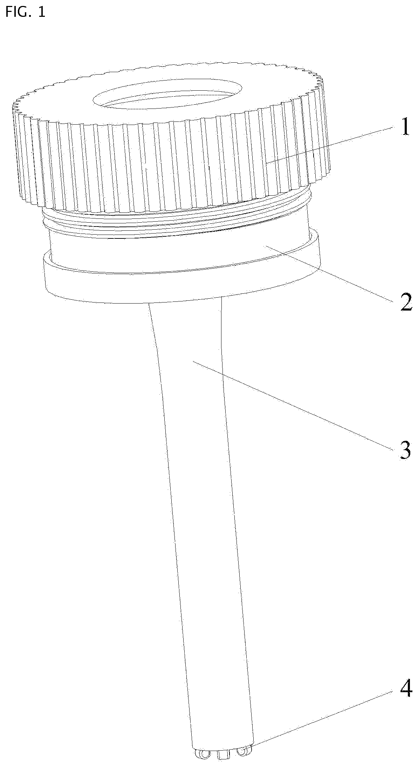

As shown in Figures 1, 2, 10, and 11, in one embodiment of the present invention, a variable diameter tissue expansion device is provided. Specifically, the variable diameter tissue expansion device includes an operating frame 2, a pushing block 5, the support rods 4, and an expansion tube 3. Both the operating frame 2 and the propulsion block 5 are equipped with a central cavity, and multiple support rods 4 are distributed in a circular array around the cavity on the operating frame 2. The support rods 4 include the first sliding part 17, the second sliding part 16, and an expansion part 18, the first sliding part 17 is in sliding fit with the operating frame 2, the second sliding part 16 is in sliding fit with the pushing block 5, and the pushing block 5 is in sliding fir with the operating frame 2. Multiple expansion parts 18 are supported on the inner wall of the expansion tube 3. wherein the angle (α) between the expansion part 18 and the first sliding part 17 is a right angle, and the angle (β) between the expansion part 18 and the second sliding part 16 is an obtuse angle, which can expand the diameter of the expansion tube 3 during sliding. The cavity of operating frame 2, the cavity of propulsion block 5, and the inner cavity of expansion tube 3 together form the working channel for surgery.

In this embodiment, a variable diameter tissue expansion device is provided, which is suitable for surgery to establish subcortical passages beneath the cortex in neurosurgery (such as intraventricular tumors, cysts). Expansion tube 3 has elasticity, and when in use, it enters the surgical site in a contracted manner, providing non-invasive access to the subcortical space. After entering the surgical position, push the pushing block 5 to slide downwards, push the second sliding part 16 to slide, and the first sliding part 17 slides on the operating frame 2. Due to the angle between the expansion part 18 and the first sliding part 17 being a right angle and the angle between the expansion part 18 and the second sliding part 16 being an obtuse angle, when the pushing block 5 slides downward, the expansion part 18 expands radially outward along the expansion tube 3, thereby enabling the expansion tube 3 to expand radially to a working channel diameter suitable for surgery. The diameter of the working channel can be adjusted at any time according to the needs during the surgical process. After the surgery is completed, the expansion tube 3 contracts when the pushing block 5 slides upwards, allowing for safe withdrawal. In this embodiment, when the expansion tube 3 enters the brain tissue, it is in a contracted state with a small outer diameter, which can reduce the extent of damage to the cerebellar tissue during insertion; when the expansion tube 3 expands radially, the compression of brain tissue in the circumferential direction is more uniform and slow, without axial frictional resistance, which can better protect brain tissue.

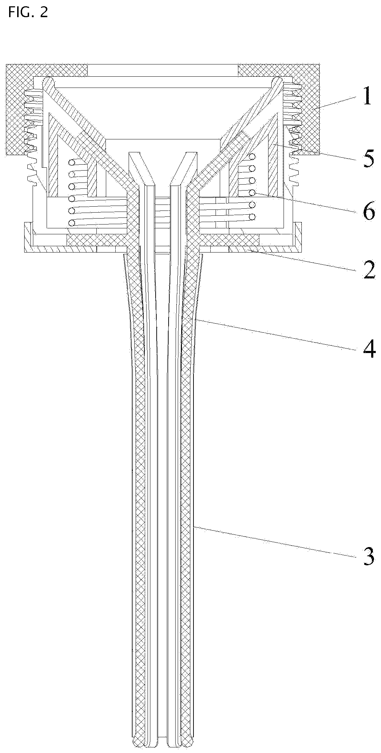

As shown in Figure 2, in one embodiment of the present invention, an elastic body 6 is also included, the elastic body 6 is installed on the operating frame 2, located between the operating frame 2 and the pushing block 5, and can provide a reset thrust for the pushing block 5, so that the pushing block 5 can slide upward under the thrust of the elastic body 6 to reset. Specifically, one end of the elastic body 6 is in contact with the operating frame 2, and another end is in contact with the pushing block 5. As an implementable solution, the elastic body 6 can be a pressure spring, a rubber block, or a combination of a pressure spring and a rubber block.

As shown in Figures 1 and 2, in one embodiment of the present invention, an adjusting knob 1 is further included, the adjusting knob 1 is equipped with a central cavity, which is used to fit with the cavity of the operating frame 2, the cavity of the pushing block 5, and the inner cavity of the expansion tube 3 to form a working channel for surgery. In this embodiment, the adjusting knob 1 is installed on the operation frame 2 and is offset against one end of the pushing block 5. Specifically, one end of the pushing block 5 is in contact with the adjusting knob 1, and another end is in contact with the elastic body 6, the pushing block 5 is located between the adjusting knob 1 and the elastic body 6. The adjusting knob 1 is connected to the operating frame 2 through threads, by adjusting the length of the thread fit, the adjusting knob 1 can be rotated up and down on the operating frame 2, thereby pushing the pushing block 5 to slide, the radial size of the expansion tube 3 can be adjusted at any time according to the needs, which is convenient to operate and can save surgical time.

Furthermore, in this embodiment, the circumference of the adjusting knob 1 is provided with anti slip patterns, which is convenient for the operator to turn the adjusting knob 1 and is more convenient for use.

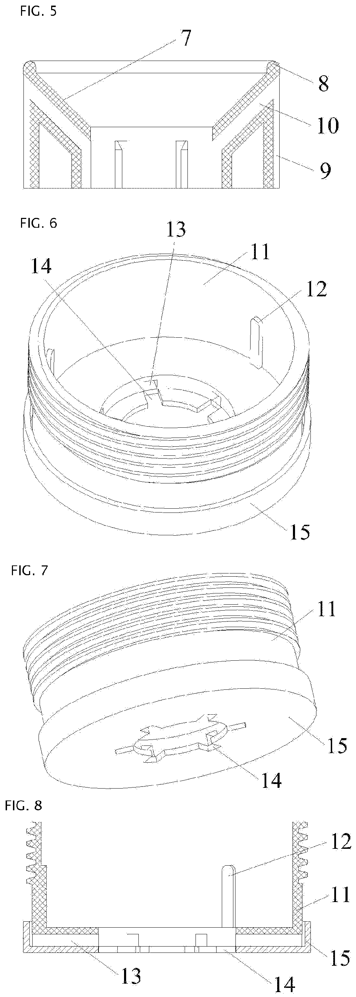

As shown in Figures 2, 6, 7, and 8, in one embodiment of the present invention, the operating frame 2 includes the first operating block 15 connectively and the second operating block 11 connectively. wherein the first operating block 15 is equipped with limiting position grooves 14, which are multiple and distributed in a circular array along the cavity of the operation frame 2. Multiple limiting position grooves 14 correspond one-to-one with multiple expansion parts 18, when the expansion parts 18 expand radially along the expansion tube 3 to the maximum size position (when the pushing block 5 slides down to the bottom), the expansion parts 18 are located exactly inside the limiting position grooves 14, furthermore, the limiting position grooves 14 can restrict the expansion part 18 from swinging in the tangential direction of the expansion tube 3, thereby improving the reliability for use.

As shown in Figures 2, 6, 7, and 8, in one embodiment of the present invention, the second operating block 11 is provided with the first sliding groove 13 that slides and fits with the first sliding portion 17, and the first sliding groove 13 is located on the bottom surface of the second operating block 11. Along the axial direction of the expansion tube 3, the depth of the first sliding groove 13 is smaller than the height of the first sliding portion 17. The first operating block 15 and the second operating block 11 are connected by threads, and there is an adjustable gap between the first operating block 15 and the second operating block 11. The sum of the adjustable gap and the depth of the first sliding groove 13 can be adjusted, when the sum of depths is greater than the height of the first sliding part 17, the first sliding part 17 can slide normally. When the sum of depths is less than the height of the first sliding part 17, the first sliding part 17 is stuck. During surgery, when it is necessary to fix the current position of the support rod 4, the support rods 4 can be locked by reducing the adjustable gap. When it is necessary to expand or contract the expansion tube 3, the support rods 4 can be unlocked by increasing the adjustable gap.

As shown in Figures 2, 6, 7, and 8, in one embodiment of the present invention, the second operating block 11 is provided with the first sliding groove 13 that slides and fits with the first sliding portion 17, and the first sliding groove 13 is located on the bottom surface of the second operating block 11. Along the axial direction of the expansion tube 3, the depth of the first sliding groove 13 is smaller than the height of the first sliding portion 17. The first operating block 15 and the second operating block 11 are connected by snap-in connection, during the snap-in connection, the first snap-in connection position and the second snap-in connection position are provided, the first operating block 15 and the second operating block 11 are provided with the first gap when the first snap-in connection position is engaged, and the first operating block 15 and the second operating block 11 provided with the second gap when the second snap-in connection position is engaged. When the snap-in cooperation is in the first snap-in connection position, the sum of the depth values of the first gap and the first sliding groove 13 is greater than the height value of the first sliding part 17, the support rods 4 can slide, and the expansion tube 3 can expand or contract; when the snap-in cooperation is in the second snap-in connection position, the sum of the depth values of the second gap and the first sliding groove 13 is less than the height value of the first sliding part 17, and the support rods 4 are locked.

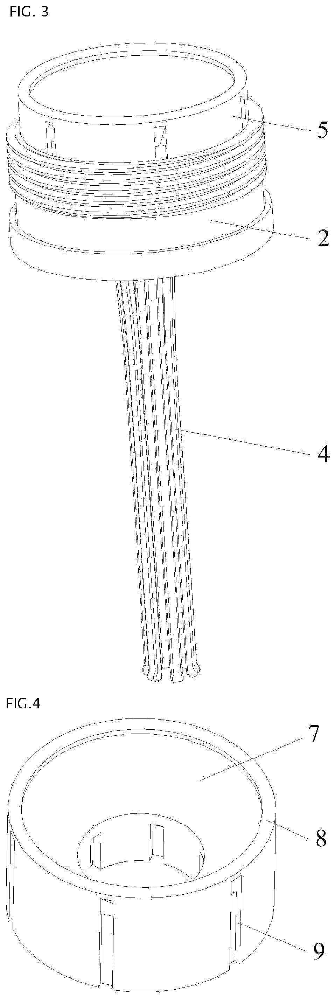

As shown in Figures 2 to 5, in one embodiment of the present invention, the pushing block 5 is provided with the second sliding groove 10 that slides and fits with the second sliding part 16, there are multiple second sliding grooves 10, which correspond one-to-one with multiple second sliding parts 16, and can drive the second sliding parts 16 to slide along the second sliding grooves 10. Preferably, the length of the second sliding groove 10 is greater than the length of the second sliding portion 16, so as not to hinder the expansion of the support rods 4.

As shown in Figures 2 to 5, in one embodiment of the present invention, the operating frame 2 is provided with a guide bar 12, and the pushing block 5 is provided with a guide groove 9, the guide bar 12 slides and fits with the guide groove 9 to provide sliding guidance for the pushing block 5, preventing the pushing block 5 and the operating frame 2 from rotating relative to each other and blocking the support rods 4. Preferably, the length of the guide groove 9 is greater than the length of the guide bar 12, ensuring that it plays a guiding role throughout the entire sliding process of the pushing block 5.

Furthermore, in this embodiment, both guide grooves 9 and guide bars 12 are multiple, and multiple guide bars 12 correspond one-to-one with multiple guide grooves 9. Multiple guide bars 12 are distributed in a circular array on the operating frame 2, and multiple guide grooves 9 are distributed in a circular array on the circumference of the pushing block 5.

As shown in Figures 2 to 5, in one embodiment of the present invention, the top of the pushing block 5 is funnel-shaped, with a sloping surface 7 and a fitting edge 8, the fitting edge 8 is located at the periphery of the top of the pushing block 5, on the outside of the sloping surface 7. The sloping surface 7 is used to avoid adjusting knob 1, and the fitting edge 8 is used to press against adjusting knob 1, which can reduce the contact area with adjusting knob 1, thereby reducing frictional resistance and facilitating adjustment. The slope surface 7 gradually slopes downwards from the periphery of the pushing block 5 towards the middle, facilitating the entry of instruments such as endoscopes, drainage tubes, surgical forceps, etc. Preferably, in this embodiment, the top surface of the fitting edge 8 is a circular arc surface, which is pressed against the adjusting knob 1 to further reduce the frictional resistance during adjustment.

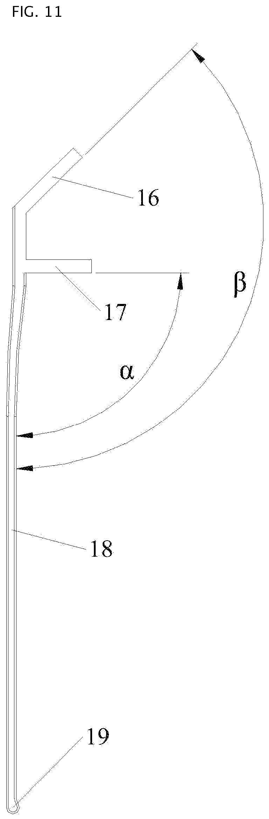

As shown in Figure 11, in one embodiment of the present invention, the bottom end of the expansion part 18 is provided with an outwardly curved snap-in protrusion 19, the snap-in protrusion 19 protrudes from the outer surface of the expansion part 18, and can prevent the expansion tube 3 from detaching from the support rods 4 during use.

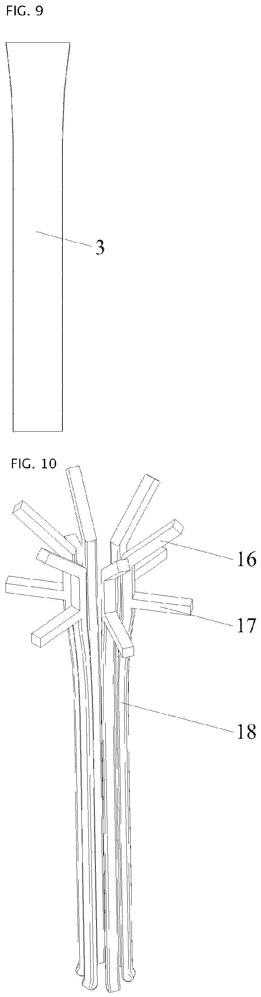

As shown in Figure 9, in one embodiment of the present invention, the top outer diameter of the expansion tube 3 is outwardly expanded along the axial direction of the expansion tube 3, that is, the top size of the expansion tube 3 is larger, while the other sizes are smaller than the top, which allows the top to play a certain supporting role during surgery and also facilitates surgical operations.

As shown in Figures 1 to 11, the working process of the present invention will be described in detail below:

The variable diameter tissue expansion device includes an operating frame 2, a pushing block 5, support rods 4, an expansion tube 3, an elastic body 6, and an adjusting knob 1. During use, the operating frame 2 is fixed, when turning the adjusting knob 1 clockwise, the adjusting knob 1 moves downwards relative to the operating frame 2, pushing the pushing block 5 to slide downwards. When the pushing block 5 slides downwards, an oblique downward force is applied to the second sliding part 16 through the second sliding groove 10, causing the second sliding part 16 to slide along the second sliding groove 10, Due to the sliding fit between the first sliding groove 13 and the first sliding part 17, under the joint action of the two sliding fits (the sliding fit between the first sliding groove 13 and the first sliding part 17, and the sliding fit between the second sliding groove 10 and the second sliding part 16), the support rods 4 expands radially outward along the expansion tube 3, promoting the expansion tube 3 to expand radially. Expansion tube 3 is made of highly ductility elastic material, such as elastic TPU (Thermoplastic Polyurethane) material or silicone material. When adjusting knob 1 is turned counterclockwise, it moves upward relative to operating frame 2, the pushing block 5 slides upward under the action of elastic body 6, and causes support rods 4 to contract radially along expansion tube 3, thereby causing expansion tube 3 to contract radially.

It is known from common technical knowledge that the present invention can be implemented through other embodiments that do not deviate from its spirit or necessary features. Therefore, the above-mentioned publicly available implementation embodiments are only examples in all aspects and are not exclusive. All changes within the scope of the present invention or within the scope equivalent to the present invention are included in the present invention.

## Claims

1. A variable diameter tissue expansion device, it is **characterized by**: the device includes:
an operating frame, a pushing block, support rods and an expansion tube;
both the operating frame and the pushing block are provided with a central cavity;
a plurality of the support rods are distributed in a circular array around the cavity on the operating frame, the support rods comprise the first sliding part, the second sliding part and an expansion part, and the first sliding part is in the sliding fit with the operating frame, the second sliding part is in the sliding fit with the pushing block, and the pushing block is in the sliding fit with the operating frame;
a plurality of expansion parts are supported on the inner wall of the expansion tube, the angle between the expansion part and the first sliding part is a right angle, and the angle between the expansion part and the second sliding part is an obtuse angle, so that the diameter of the expansion tube can be expanded during sliding.

2. The variable diameter tissue expansion device according to claim 1, **characterized in that**: further comprising an elastic body installed on the operating frame, located between the operating frame and the pushing block;
one end of the elastic body is in contact with the operating frame, and another end is in contact with the pushing block, used to provide reset thrust for the pushing block;
the elastic body is either a pressure spring, a rubber block, or a combination thereof.

3. The variable diameter tissue expansion device according to claim 2, **characterized in that**: further comprising an adjusting knob, wherein the adjusting knob is provided with a central cavity;
the adjusting knob is installed on the operating frame and is in contact with the pushing block, which is located between the adjusting knob and the elastic body;
the adjusting knob is threaded with the operating frame to push the pushing block to slide when rotated.

4. The variable diameter tissue expansion device according to claim 1, **characterized in that**: the operating frame comprises the first operating block connectively and the second operating block connectively;
the first operating block is equipped with the limiting position groove, which is multiple; multiple limiting position grooves correspond one-to-one with multiple expansion parts, used to limit the swing of the expansion part when it reaches its maximum position.

5. The variable diameter tissue expansion device according to claim 4, **characterized in that**: the second operating block is provided with the first sliding groove that slides and fits with the first sliding part, the first sliding groove is located on the bottom surface of the second operating block;
along the axial direction of the expansion tube, the depth of the first sliding groove is less than the height of the first sliding part;
the first operating block and the second operating block are threaded together, there is an adjustable gap between the first operating block and the second operating block, the adjustable gap is used to fit with the first sliding groove to slide or lock the first sliding part.

6. The variable diameter tissue expansion device according to claim 4, **characterized in that**: the second operating block is provided with the first sliding groove that slides and fits with the first sliding part, the first sliding groove is located on the bottom surface of the second operating block;
along the axial direction of the expansion tube, the depth of the first sliding groove is less than the height of the first sliding part;
the first operating block and the second operating block are connected by snap-in connection, and are provided with the first snap-in connection position and the second snap-in connection position, the first operating block and the second operating block are provided with the first gap when the first the second snap-in connection position is engaged, the first operating block and the second operating block are provided with the second gap when the second snap-in connection position is engaged;
the sum of the depth values of the first gap and the first sliding groove is greater than the height value of the first sliding part, the sum of the depth values of the second gap and the first sliding groove is less than the height value of the first sliding part.

7. The variable diameter tissue expansion device according to claim 1, **characterized in that**: the operating frame is equipped with guide bars, and the pushing block is equipped with guide grooves, the guide bars and the guide grooves slide together to provide sliding guidance for the pushing block;
the length of the guide groove is greater than the length of the guide bars;
the guide grooves and guide bars are both multiple, and multiple guide bars correspond one-to-one with multiple guide grooves.

8. The variable diameter tissue expansion device according to claim 3, **characterized in that**: the top of the pushing block is funnel-shaped, with a sloping surface and a fitting edge;
the sloping surface gradually slopes downwards from the periphery of the pushing block towards the middle;
the fitting edge is set at the periphery of the top of the pushing block, located on the outer side of the sloping surface;
the top surface of the fitting edge is a circular arc surface, used to rest against the adjusting knob;
the pushing block is equipped with the second sliding groove that slides and fits with the second sliding part, the second sliding grooves are multiple, the second multiple sliding grooves correspond one-to-one with the second multiple sliding parts;
the length of the second sliding grooves are greater than the length of the second sliding part.

9. The variable diameter tissue expansion device according to claim 1, **characterized in that**: the bottom end of the expansion part is provided with an outwardly curved snap-in protrusion, the snap-in protrusion protrudes from the outer surface of the expansion part and is used to prevent the expansion tube from detaching from the support rods.

10. The variable diameter tissue expansion device according to claim 1, **characterized in that**: along the axial direction of the expansion tube, the top outer diameter of the expansion tube is outwardly expanded.
